# DEMANDE DE BREVET EUROPEEN

(11) **EP 1 430 934 A1**
(43) Date de publication de la demande: **23.06.2004**
(21) Numéro de dépôt: 04075491.3
(22) Date de dépôt: 08.11.2000
(51) Int. Cl.: A61P 35/00, A61K 31/4985

(54) **Produit comprenant un inhibiteur de la transduction des signaux des protéines G hétérotrimetriques en association avec un autre agent anti-cancéreux pour une utilisation thérapeutique dans le traitement du cancer**

(30) Priorité: 09.11.1999 FR 9914037; 06.01.2000 FR 0000104
(62) Demande divisionnaire de: 00976116.4
(71) Demandeur: Société de Conseils de Recherches et d'Applications Scientifiques ( S.C.R.A.S.), 75016 Paris (FR)
(72) Inventeur: Prevost, Grégoire, 92160 Antony (FR); Lonchampt, Marie Odile, 94550 Chevilly-Larue (FR); Gordon, Thomas, Medway MA 02053 (US); Morgan, Barry, Franklin MA 02038 (US)
(74) Mandataire: Bourgouin, André

(57) **Abrégé**

L'invention concerne un produit comprenant au moins un inhibiteur de la transduction des signaux des protéines G hétérotrimériques, en association avec au moins un autre agent anti-cancéreux de type agent alkylant, pour une utilisation thérapeutique simultanée, séparée ou étalée dans le temps, dans le traitement du cancer.

## Description

La description de la demande divisionnaire est identique à celle de la demande n° EP 00976116.4 (qui correspond à la demande PCT/FR00/03098). Seules les revendications sont différentes, basées sur la description comme détaillé ci-après :

### Revendication 1 :

Les bases utilisées pour rédiger cette revendication se trouvent :
- pour les composés de formule générale (I), dans le passage page 4, ligne 3 à page 5, ligne 23 pris avec les préférences indiquées en page 6, lignes 7 à 10 et la définition des termes alkyle inférieur et alkyle données en page 5, lignes 24 à 27 et page 13, lignes 9 à 14;
- pour les agents alkylants, en page 4, ligne 4 ou page 15, ligne 1.

### Revendication 2 :

Les bases pour les caractéristiques supplémentaires de cette revendication dépendante se trouvent en page 4, ligne 4 ou page 15, ligne 1.

### Revendication 3 :

Les bases pour les caractéristiques supplémentaires de cette revendication dépendante peuvent être trouvées dans les exemples de composés préférés mentionnés dans le passage allant de la page 6, ligne 16 à la page 7, ligne 13 qui répondent à la formule générale **(I**_{**A**}**).**

### Revendication 4 :

Les bases pour les caractéristiques supplémentaires de cette revendication dépendante se trouvent en page 16, lignes 14 à 21.

### Revendication 5 :

Les bases pour les caractéristiques supplémentaires de cette revendication dépendante se trouvent en page 17, lignes 13 à 26.

### Revendication 6 :

Les bases pour cette revendication se trouvent page 17, lignes 27-28.

Compte tenu des éléments évoqués ci-dessus, nous considérons que la condition visée à l'art. 76(1), 2^{e} phrase CBE est bien respectée et que la présente demande doit par conséquent bénéficier de la date de dépôt de la demande initiale.

La présente invention concerne un produit comprenant au moins un inhibiteur de la transduction des signaux des protéines G hétérotrimériques, celui-ci répondant de préférence à la formule générale **(I)** définie plus loin, en association avec au moins un autre agent anti-cancéreux, de préférence choisi parmi le groupe composé du taxol, des analogues du taxol, de la gemcitabine et des inhibiteurs de prényltransférases, particulièrement les composés de formules générales **(II)** ou **(III)** définies plus loin, pour une utilisation thérapeutique simultanée, séparée ou étalée dans le temps, dans le traitement du cancer.

Le développement des nouveaux traitements anti-cancéreux passent en grande partie par la découverte d'associations efficaces entre différentes classes thérapeutiques pour accentuer l'effet antitumoral de chaque classe.

L'association entre l'anticorps anti-Her-2/neu et le cis-platine ou l'étoposide inhibe la prolifération des cellules tumorales mammaires de manière plus importante que la simple addition des effets de chaque produit (cf. Pegram, M., et coll., *Oncogene,* **18**(1999): 2241-2251). L'association de cet anticorps avec le taxol ou le méthotrexate montre une addition des effets alors que son association avec le 5-fluorouracyle montre un antagonisme des produits (McGuire W.P. et coll., *Semin. Oncol*. **1997** Feb. 24 (1 Suppl 2):S2-13-S2-16).

Les inhibiteurs de famésyltransférases agissent en synergie avec des agents qui dépolymérisent les microtubules (taxol, épothilones) (cf. Moasser et coll., *Proc. Natl*. *Acad. Sci. U.S.A.* (1998), **95,** 1369-1374). Les associations d'inhibiteurs de farnésyltransférases avec les cytotoxiques doxorubicine, cisplatine ou 5-fluorouracyle montrent seulement une addition des effets.

Les protéines G hétérotrimériques sont, en fait, l'association structurale de trois sous-unités distinctes appelées α, β et γ, mais fonctionnent comme des entités dissociables constituées par des sous-unités α d'un côté et des dimères β/γ de l'autre. Différentes formes de sous-unités de type α, β et γ sont décrites.

Les protéines G participent à la transmission de signaux de l'extérieur de la cellule grâce à son interaction avec les récepteurs à sept domaines transmembranaires vers l'intérieur par l'intermédiaire de différents effecteurs incluant l'adénylate cyclase, la phospholipase C ou encore les canaux ioniques. L'enzyme adénylate cyclase génère de l'adénosine monophosphate cyclique (AMPc) (cf. Gilman, *Biosci. Rep*. (1995), **15**, 65-97). Ainsi, on sait que pour activer l'adénylate cyclase, il est nécessaire que les protéines G soient transitoirement dans une forme hétérotrimérique, forme dans laquelle le monomère constitué par une sous-unité α est associé au dimère constitué par les sous-unités β et γ. On sait encore que pour que les protéine G se trouvent dans leur forme hétérotrimérique, il faut qu'elles soient fixées par leurs sous-unités γ à la membrane. C'est uniquement dans cette situation que le signal de l'extérieur de la cellule peut activer la sous-unité α d'une protéine G, laquelle pourra, après dissociation, moduler les effecteurs comme l'adénylate cyclase et moduler la production d'AMPc.

On sait aussi que les dimères β/γ peuvent activer directement des effecteurs conduisant à l'activation de kinases régulées par des signaux extracellulaires (ERKs) ou des MAP kinases. Un lien direct entre les sous-unités β/γ et les kinases src ou src like a été démontré (cf. Gutkind, *J*. *Biol. Chem*. (1998), **273**, 1839-1842).

Les effets néfastes d'un taux anormal d'AMPc sont également connus et ont notamment lieu au niveau des fonctions biologiques ou désordres suivants : odorat, goût, perception de la lumière, neurotransmission, neurodégénérescence, fonctionnement des glandes endocrines et exocrines, régulations autocrine et paracrine, tension artérielle, embryogénèse, prolifération cellulaire bénigne, oncogénèse, infection virale et fonctions immunologiques, diabète et obésité.

La demanderesse avait elle-même déjà décrit dans la demande de brevet PCT WO 00/02881 l'utilisation des composés de formule générale **(I)** telle que définie ci-après en tant qu'inhibiteurs de protéine G. Certains de ces produits avaient été décrits auparavant dans la demande de brevet PCT WO 97/30053.

Les inhibiteurs de prényltransférases sont déjà utilisés dans le domaine du traitement du cancer (cf. Sebti et coll., *Pharmacol. Ther.* (1997), **74,** 103-114 ; Sepp-Lorenzino et coll., *Cancer Res.* (1997), **55,** 5302-5309). L'utilité des inhibiteurs de prényltransférases dans ce type de traitement proviendrait de leur action qui empêcherait la prénylation au niveau du substrat Ras. Cependant, la prénylation de certaines formes de Ras n'est pas modifiée par les inhibiteurs de prénylation (Lemer et coll., *Oncogene* (1997), **15,** 1283-1288).

En ce qui concerne les agents anti-cancéreux, des inhibiteurs de prényltransférases sont notamment décrits dans les demandes de brevet suivantes : demandes PCT WO 97/21701, WO 97/16443, WO 98/00409, WO 96/21456, WO 97/24378, WO 97/17321, WO 97/18813, WO 95/00497; brevets US 5,532,359, US 5,523,430, US 5,510,510 et US 5,627,202. Par ailleurs, les composés de formule générale **(II)** ont été décrits dans la demande de brevet PCT WO 00/39130. Le taxol est quant à lui notamment décrit dans *Merck Index,* 11th ed., 1989, sous le numéro de rubrique 9049 et dans les références citées. Des analogues de camptothécines ont notamment été décrits dans le brevet US 4,894,456 et dans les demandes de brevet PCT WO 94/11376, WO 97/00876, WO 98/28304, WO 98/28305, WO 99/11646 et WO 99/33829.

Un produit selon l'invention offre l'avantage de pouvoir utiliser des doses moins élevées des agents anti-cancéreux choisis, ce qui a pour principal effet de diminuer la toxicité du traitement tout en obtenant un effet pharmacologique au minimum additif.

L'invention a donc pour objet un produit comprenant au moins un inhibiteur de la transduction des signaux des protéines G hétérotrimériques en association avec au moins un agent anti-cancéreux, de préférence choisi parmi le groupe composé du taxol, des analogues du taxol, de la gemcitabine et des inhibiteurs de prényltransférases, pour une utilisation thérapeutique simultanée, séparée ou étalée dans le temps, dans le traitement du cancer.

De préférence, l'inhibiteur de prényltransférases associé à l'inhibiteur de la transduction des signaux des protéines G hétérotrimériques sera un inhibiteur de farnésyltransférases.

Bien que le taxol, les analogues du taxol, la gemcitabine et les inhibiteurs de prényltransférases, soient préférés, de nombreux autres agents anti-cancéreux peuvent être également associés, selon l'invention, à un inhibiteur de la transduction des signaux des protéines G hétérotrimériques, par exemple : des inhibiteurs enzymatiques comme les inhibiteurs de topoisomérases comme la campthotécine et les analogues de la camptothécine (sous forme d'analogues comportant un cycle lactonique E à six chaînons tels par exemple les composés décrits dans la demande de brevet PCT WO 94/11376, sous forme d'analogues comportant un cycle lactonique E à sept chaînons tels par exemple les composés décrits dans la demande de brevet PCT WO 97/00876 ou encore sous forme d'analogues tétracycliques ouverts tels par exemple les composés décrits dans la demande de brevet PCT WO 99/33829), les inhibiteurs de phosphatases Cdc25, les inhibiteurs de MAP kinases ou de MAP kinases kinases, les inhibiteurs de protéine kinase C, les inhibiteurs de tyrosine kinases, les inhibiteurs de télomérases ; des inducteurs d'apoptose ; des agents alkylants comme le cis-platine ; des agents anti-métaboliques comme le 5-fluorouracile ; des agents de différentiation ; des poisons du fuseau cellulaire ; des inhibiteurs d'angiogénèse ; des anti-hormones ou des antagonistes des récepteurs stéroïdiens ; des anti-oxydants ; des agents anti-sens ; des agents anti-p53 (thérapie génique) ; des agents de chémoprévention ; des agents anti-viraux ; des agents immunothérapeutiques ; des anti-corps comme l'héréguline.

De préférence, l'inhibiteur de la transduction des signaux des protéines G hétérotrimériques sera un composé de formule générale **(I)** 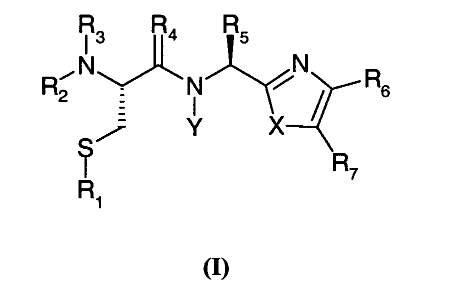 correspondant aux sous-formules **(I**_{**A**}**)** ou **(I**_{**B**}**)** : 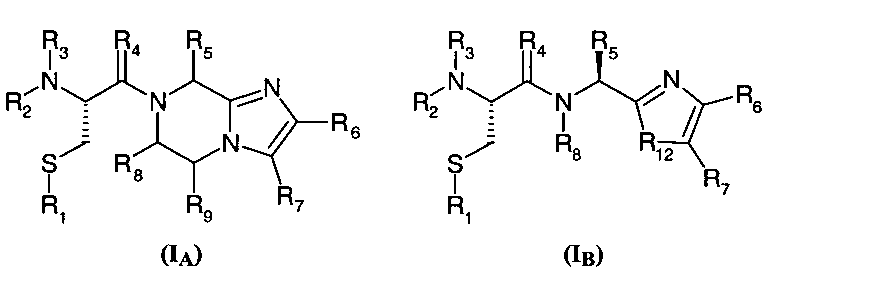 dans lesquelles :
X représente R₁₂ et Y représente R₈, ou X et Y complètent un cycle à 6 chaînons, l'ensemble X-Y représentant le radical -CH(R₈)-CH(R₉)- ;
R₁ représente H, un radical alkyle ou alkylthio ;
R₂ et R₃ représentent indépendamment H ou un radical alkyle ;
R₄ représente H₂ ou O ;
R₅ représente H, ou l'un des radicaux alkyle, alkényle, alkynyle, aryle, aralkyle, hétérocyclyle ou hétérocyclylalkyle, ces radicaux pouvant éventuellement être substitués par des radicaux choisis parmi le groupe composé d'un radical alkyle, -O-R₁₀, -S(O)ₘR₁₀ (m représentant 0, 1, ou 2), -N(R₁₀)(R₁₁), -N-C(O)-R₁₀, -NH-(SO₂)-R₁₀, -CO₂-R₁₀, C(O)-N(R₁₀)(R₁₁), et -(SO₂)-N(R₁₀)(R₁₁) ;
R₆ et R₇ représentent indépendamment H, un radical -C(O)-NH-CHR₁₃-CO₂R₁₄, ou l'un des radicaux alkyle, aryle, aralkyle, hétérocyclyle ou hétérocyclylalkyle, ces radicaux pouvant éventuellement être substitués par des radicaux choisis parmi le groupe composé des radicaux OH, alkyle ou alkoxy, N(R₁₀)(R₁₁), COOH, CON(R₁₀)(R₁₁), et halo,
   ou R₆ et R₇ forment ensemble un radical aryle ou un hétérocycle ;
R₈ et R₉ représentent indépendamment, H, ou l'un des radicaux alkyle, aryle, aralkyle, hétérocyclyle ou hétérocyclylalkyle, ces radicaux pouvant éventuellement être substitués par des radicaux choisis parmi le groupe composé des radicaux OH, alkyle ou alkoxy, N(R₁₀)(R₁₁), COOH, CON(R₁₀)(R₁₁) et halo,
   ou R₈ et R₉ forment ensemble un radical aryle ou un hétérocycle ;
R₁₀ et R₁₁, représentent indépendamment H, un radical aryle ou hétérocyclyle, ou un radical alkyle, aralkyle ou hétérocyclylalkyle ;
R₁₂ représente NR₉, S, ou O ;
R₁₃ représente un radical alkyle éventuellement substitué par un radical choisi parmi les radicaux alkyle, -OR₁₀, -S(O)ₘR₁₀ (m représentant 0, 1, ou 2) et -N(R₁₀)(R₁₁) ;
R₁₄ représente H ou un radical alkyle ;
ou un sel pharmaceutiquement acceptable d'un tel composé.

Par radical alkyle inférieur, on entend un radical alkyle linéaire ou ramifié comptant de 1 à 6 atomes de carbone, et notamment les radicaux méthyle, éthyle, propyle, isopropyle, butyle, isobutyle, sec-butyle et tert-butyle, pentyle, néopentyle, isopentyle, hexyle, isohexyle. Par radical hétérocycle, on entend un radical constitué de un ou plusieurs cycles et incluant au moins un hétéroatome. Par radical arylalkyle, hétérocycle alkyle, alkylthio ou alkoxy inférieur, on entend les radicaux dont le radical alkyle a la signification indiquée précédemment.

De préférence, les composés de formule générale **(I)** seront tels que :
X et Y complètent un cycle à 6 chaînons, l'ensemble X-Y représentant le radical -CH(R₈)-CH(R₉)- ;
R₁ représente un radical alkyle ou inférieur ;
R₂ et R₃ représentent H ;
R₄ représente O ;
R₅ représente H, ou l'un des radicaux alkyle inférieur, cycloalkyle, cycloalkylalkyle, , arylsulfonylalkyle inférieur, aralkoxyalkyle inférieur, ces radicaux pouvant éventuellement être substitués par des radicaux choisis parmi le groupe composé d'un radical alkyle inférieur ou -O-R₁₀ ;
R₆ et R₇ représentent indépendamment H ou un radical aryle éventuellement substitué par des radicaux choisis parmi le groupe composé des radicaux OH, alkyle ou alkoxy inférieur,
R₈ et R₉ représentent H ;
et R₁₀ et R₁₁, représentent indépendamment H ou un radical alkyle inférieur.

Sont en particulier préférés pour l'invention les composés de formule générale **(I)** suivants :
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la 7-(2-amino-1-oxo-3-thiopropyl)-8-butyl-2-(2-méthoxyphényl)-5,6,7,8-tétrahydroimidazo[1,2a]pyrazine ;
- le disulfure de bis-1,1'-[7-(2-amino-1-oxo-3-thiopropyl)-2-(2-méthoxyphényl)-8-(1-méthylpropyl)-5,6,7,8-tétrahydroimidazo[1,2a]pyrazine] ;
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine ;
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) ;
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
- la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydro-imidazo[1,2a]pyrazine, ou sa forme dimérique ;
- et la 7-(2-amino-1-oxo-3-thiopropyl)-2-(2-méthoxyphényl)-8-(phénylsulfonyléthyl)-5,6,7,8-tétrahydro-imidazo[1,2a]pyrazine ;
ou un sel pharmaceutiquement acceptable d'un de ces derniers.

De préférence, lorsque l'agent anti-cancéreux associé à l'inhibiteur de la transduction des signaux des protéines G hétérotrimériques sera un inhibiteur de prényltransférases, il s'agira d'un inhibiteur de farnésyltransférases.

Plus préférentiellement, l'inhibiteur de farnésyltransférase sera choisi parmi le groupe composé :
- d'un composé de formule générale **(II)** 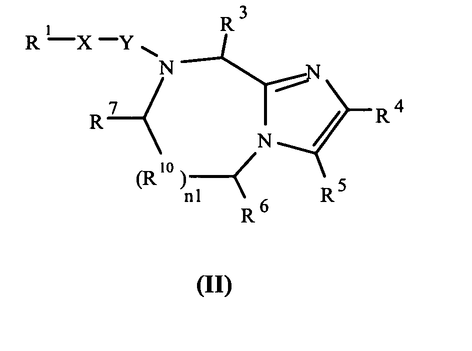 dans laquelle :
   n1 représente 0 ou 1;
   X représente, indépendamment chaque fois qu'il intervient, (CHR¹¹)ₙ₃(CH₂)ₙ₄Z(CH₂)ₙ₅ ;
   Z représentant O, N(R¹²), S, ou une liaison ;
   n3 représentant, indépendamment chaque fois qu'il intervient, 0 or 1;
   chacun de n4 et n5 représentant, indépendamment chaque fois qu'ils intervient, 0, 1, 2, ou 3 ;
   Y représente, indépendamment chaque fois qu'il intervient, CO, CH₂, CS, ou une liaison ;
   R¹ représente l'un des radicaux 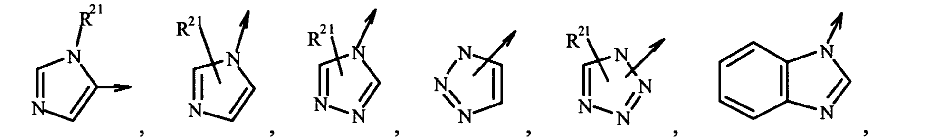 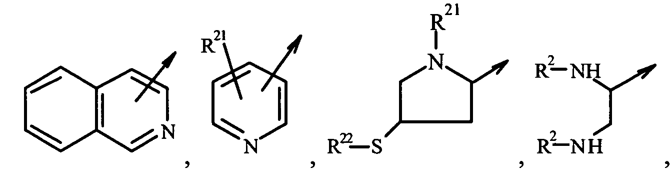 ou N(R²⁴R²⁵) ;
      chacun de R² , R¹¹, et R¹² représentant, indépendamment chaque fois qu'il intervient, H ou un radical optionnellement substitué choisi parmi le groupe consistant en un radical (C₁₋₆)alkyle et un radical aryle, ledit radical optionnellement substitué étant optionnellement substitué par au moins un radical choisi parmi les radicaux R⁸ et R³⁰, chaque substituant étant choisi indépendamment des autres ;
   R³ représente, indépendamment chaque fois qu'il intervient, H ou un radical optionnellement substitué choisi parmi le groupe consistant en les radicaux (C₁₋₆)alkyle, (C₂₋₆)alkényle, (C₂₋₆)alkynyle, (C₃₋₆)cycloalkyle, (C₃₋₆)cycloalkyl(C₁₋₆)alkyle, (C₅₋₇)cycloalkényle, (C₅₋₇)cycloalkényl(C₁₋₆)alkyle, aryle, aryl(C₁₋₆)alkyle, hétérocyclyle, et hétérocyclyl(C₁₋₆)alkyle, ledit radical optionnellement substitué étant optionnellement substitué par au moins un radical choisi parmi les radicaux R³⁰, chaque substituant étant choisi indépendamment des autres ;
   chacun de R⁴ et R⁵ représente, indépendamment chaque fois qu'il intervient, H ou un radical optionnellement substitué choisi parmi le groupe consistant en les radicaux (C₁₋₆)alkyle, (C₃₋₆)cycloalkyle, aryle et hétérocyclyle, ledit radical optionnellement substitué étant optionnellement substitué par au moins un radical choisi parmi les radicaux R³⁰, chaque substituant étant choisi indépendamment des autres, ou R⁴ et R⁵ pris ensemble avec les atomes de carbone auxquels ils sont attachés forment ensemble un radical aryle ;
   R⁶ représente, indépendamment chaque fois qu'il intervient, H ou un radical optionnellement substitué choisi parmi le groupe consistant en les radicaux (C₁₋₆)alkyle, (C₂₋₆)alkényle, (C₃₋₆)cycloalkyle, (C₃₋₆)cycloalkyl(C₁₋₆)alkyle, (C₅₋₇)cycloalkényle, (C₅₋ ₇)cycloalkényl(C₁₋₆)alkyle, aryle, aryl(C₁₋₆)alkyle, hétérocyclyle et hétérocyclyl(C₁₋₆)alkyle, ledit radical optionnellement substitué étant optionnellement substitué par au moins un radical choisi parmi les radicaux OH, (C₁₋₆)alkyle, (C₁₋₆)alkoxy, -N(R⁸R⁹), -COOH, -CON(R⁸R⁹) et halo, chaque substituant étant choisi indépendamment des autres ;
   R⁷ représente, indépendamment chaque fois qu'il intervient, H, =O, =S, H ou un radical optionnellement substitué choisi parmi le groupe consistant en les radicaux (C₁₋₆)alkyle, (C₂₋₆)alkényle, (C₃₋₆)cycloalkyle, (C₃₋₆)cycloalkyl(C₁₋₆)alkyle, (C₅₋₇)cycloalkényle, (C₅₋₇)cycloalkényl(C₁₋₆)alkyle, aryle, aryl(C₁₋₆)alkyle, hétérocyclyle et hétérocyclyl(C₁₋₆)alkyle, ledit radical optionnellement substitué étant optionnellement substitué par au moins un radical choisi parmi les radicaux OH, (C₁₋₆)alkyle, (C₁₋₆)alkoxy, -N(R⁸R⁹), -COOH, -CON(R⁸R⁹) et halo, chaque substituant étant choisi indépendamment des autres ;
   chacun de R⁸ et R⁹ représentant, indépendamment chaque fois qu'il intervient, H, (C₁₋₆)alkyle, (C₂₋₆)alkényle, (C₂₋₆)alkynyle, aryle, or aryl(C₁₋₆)alkyle ;
   R¹⁰ représente C ;
   ou bien, lorsque n1 = 0, R⁶ and R⁷ peuvent être pris ensemble avec les atomes de carbone auxquels ils sont attachés pour former un radical aryle ou cyclohexyle ;
   R²¹ représente, indépendamment chaque fois qu'il intervient, H ou un radical optionnellement substitué choisi parmi le groupe consistant en les radicaux (C₁₋₆)alkyle et aryl(C₁₋₆)alkyle, ledit radical optionnellement substitué étant optionnellement substitué par au moins un radical choisi parmi les radicaux R⁸ et R³⁰, chaque substituant étant choisi indépendamment des autres ;
   R²² représente H, (C₁₋₆)alkylthio, (C₃₋₆)cycloalkylthio, R⁸-CO-, ou un substituant de formule 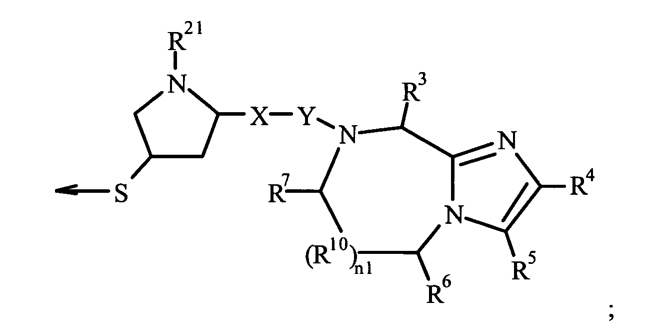 chacun de R²⁴ et R²⁵ représente, indépendamment chaque fois qu'il intervient, H, (C₁₋₆)alkyle ou aryl(C₁₋₆)alkyle ;
   R³⁰ représente, indépendamment chaque fois qu'il intervient, (C₁₋₆)alkyle, -O-R⁸, -S(O)ₙ₆R⁸, -S(O)ₙ₇N(R⁸R⁹), -N(R⁸R⁹), -CN, -NO₂, -CO₂R⁸, -CON(R⁸R⁹), -NCO-R⁸, ou halogène,
   chacun de n6 et n7 représentant, indépendamment chaque fois qu'il intervient, 0, 1 ou 2;
   ledit radical hétérocyclyle étant azépinyle, benzimidazolyle, benzisoxazolyle, benzofurazanyle, benzopyranyle, benzothiopyranyle, benzofuryle, benzothiazolyle, benzothiényle, benzoxazolyle, chromanyle, cinnolinyle, dihydrobenzofuryle, dihydrobenzothiényle, dihydrobenzothiopyranyle, dihydrobenzothio-pyranyl sulfone, furyle, imidazolidinyle, imidazolinyle, imidazolyle, indolinyle, indolyle, isochromanyle, isoindolinyle, isoquinolinyle, isothiazolidinyle, isothiazolyle, isothiazolidinyle, morpholinyle, naphthyridinyle, oxadiazolyle, 2-oxoazépinyle, 2-oxopipérazinyle, 2-oxopipéridinyle, 2-oxopyrrolidinyle, pipéridyle, pipérazinyle, pyridyle, pyridyl-N-oxyde, quinoxalinyle, tétrahydrofuryle, tétrahydroisoquinolinyle, tétrahydro-quinolinyle, thiamorpholinyle, thiamorpholinyle sulfoxyde, thiazolyle, thiazolinyle, thiénofuryle, thiénothiényle ou thiényle ;
   ledit radical aryle étant phényle ou naphthyle ;
   étant entendu que :
   lorsque n1 = 1, R¹⁰ est C et R⁶ représente H, alors R¹⁰ et R⁷ peuvent former, pris ensemble, le radical 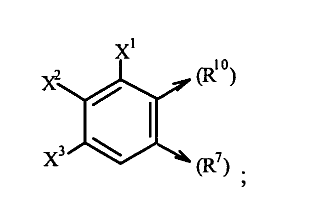
   ou lorsque n1 = 1, R¹⁰ est C, et R⁷ est =O, -H, ou =S, alors R¹⁰ et R⁶ peuvent former, pris ensemble, le radical 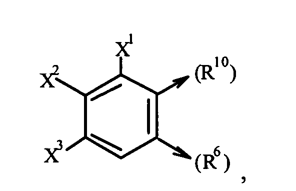
   avec chacun de X¹, X², et X³ représentant, indépendamment, H, un atome halogène, -NO₂, -NCO-R⁸, -CO₂R⁸, -CN, ou -CON(R⁸R⁹); et
   lorsque R¹ est N(R²⁴R²⁵), alors n3 représente 1, chacun de n4 et n5 représente 0, Z est une liaison, et R³ et R¹¹ peuvent former, pris ensemble, le radical 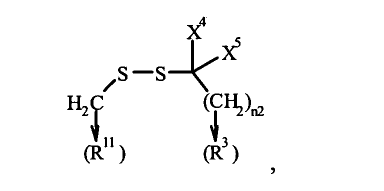
   avec n2 représentant un entier de 1 à 6, et chacun de X⁴ et X⁵ représentant, indépendamment, H, (C₁₋₆)alkyle ou aryle, ou X⁴ et X⁵ formant, pris ensemble, un radical (C₃₋₆)cycloalkyle ;
- d'un composé de formule générale **(III)** 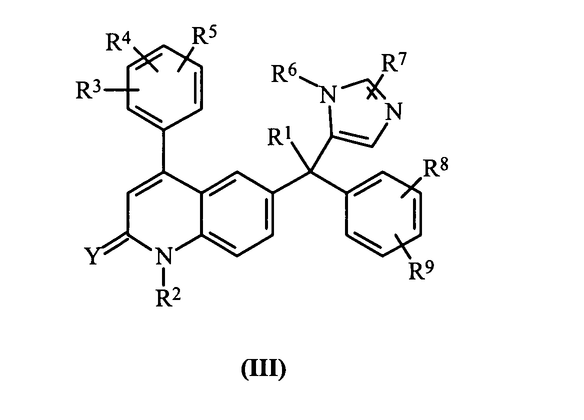 dans laquelle :
   R¹ représente H ou un radical alkyle, OR¹⁰, SR¹⁰ ou NR¹¹R¹² ;
   R² représente H ou un radical alkyle ;
   R³, R⁴ et R⁵ représentent, indépendamment, H, un atome halogène ou un radical alkyle, trihalométhyle, hydroxy, cyano ou alkoxy ;
   R⁶ représente H ou un radical alkyle ;
   R⁷ représente H, un atome halogène ou un radical alkyle, hydroxyalkyle, amino, hydroxycarbonyle ;
   R⁸ et R⁹ représentent, indépendamment, H, un atome halogène ou un radical cyano, alkyle, trihalométhyle, alkoxy, alkylthio ou dialkylamino ;
   R¹⁰ représente H ou un radical alkyle ou alkylcarbonyle ;
   R¹¹ représente H ou un radical alkyle ;
   R¹² représente H ou un radical alkyle ou alkylcarbonyle ;
   et Y représente O ou S ;
- et d'un sel pharmaceutiquement acceptable d'un composé de formule générale **(II)** ou d'un composé de formule générale **(III).**

Dans certains cas, des composés entrant dans la composition d'un produit selon la présente invention peuvent comporter des atomes de carbone asymétriques. Par conséquent, lesdits composés ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Par alkyle, lorsqu'il n'est pas donné plus de précision, on entend un radical alkyle linéaire ou ramifié comptant de 1 à 6 atomes de carbone. Par radicaux alkylcarbonyle, alkylthio, alkoxy, alkylamino, dialkylamino, alkényle, alkynyle, aralkyle, hétérocyclylalkyle, on entend respectivement les radicaux alkylcarbonyle, alkylthio, alkoxy, alkylamino, dialkylamino, alkényle, alkynyle, aralkyle, hétérocyclylalkyle dont le radical alkyle a la signification indiquée précédemment.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Enfin, par halogène, on entend les atomes de fluor, de chlore, de brome ou d'iode.

Lorsqu'une structure chimique telle qu'utilisée ici possède une flèche émanant d'elle, la flèche indique le point d'attachement. Par exemple, la structure 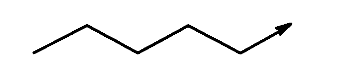 est un radical pentyle. Lorsqu'une valeur entre parenthèses apparaît près de la flèche, la valeur indique où le point d'attachement peut être trouvé dans le composé. Par exemple, dans la formule générale **(II)** 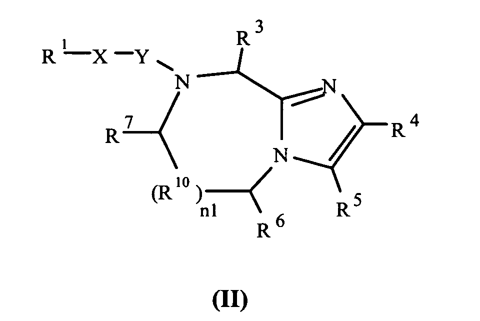 telle que définie précédemment, lorsque R¹⁰ et R⁷ sont pris ensemble pour former le radical 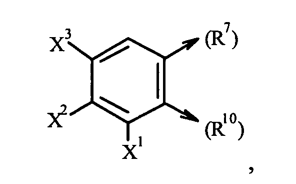 la structure suivante en résulte :

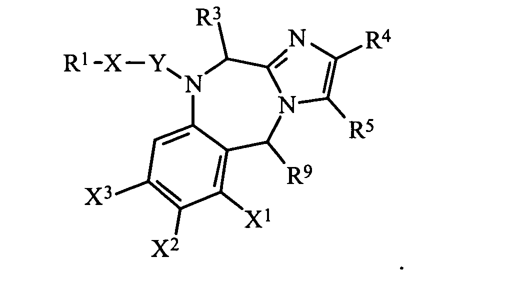

Enfin, par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J*. *Pharm.* (1986), **33,** 201-217.

En particulier, les inhibiteurs de la transduction des signaux des protéines G hétérotrimériques pourront, selon l'invention, être associés avantageusement aux composés suivants :
- des inhibiteurs de prényltransférases, et notamment des inhibiteurs de farnésyltransférases comme la 1-(2-(1-((4-cyano)phénylméthyl)imidazol-4-yl)-1-oxoéthyl-2,5-dihydro-4-(2-méthoxyphényl)imidazo[1,2c][1,4]benzodiazépine, la 4-(2-bromophényl)-1-(2-(1-((4-cyano-3-méthoxy)phénylméthyl)-imidazo-5-yl)-1-oxoéthyl)-1,2-dihydro-8-fluoro-imidazo[1,2a][1,4]-benzodiazépine ou la (±)-4-(3-chlorophényl)-6-[(4-chlorophényl)-amino-(1-méthyl-1H-imidazol-5-yl)méthyl]-1-méthyl-2(1H)quinolinone ;
- des poisons du fuseau cellulaire comme le taxol ;
- des agents alkylants comme la cisplatine ;
- des agents anti-métaboliques comme la gemcitabine ou le 5-fluorouracyle.

De préférence, les inhibiteurs de la transduction des signaux des protéines G hétérotrimériques employés pour l'invention seront tels qu'ils correspondent à la sous-formule générale **(I**_{**A**}**)** telle que définie précédemment dans laquelle :
R₁ représente H ;
R₂ et R₃ représentent indépendamment H ou un radical alkyle inférieur ;
R₄ représente O ;
R₅ représente H, ou l'un des radicaux alkyle inférieur, cycloalkyle ou cycloalkylalkyle ;
R₆ représente un radical aryle éventuellement substitué par des radicaux choisis parmi le groupe composé des radicaux OH, alkyle ou alkoxy inférieur, N(R₁₀)(R₁₁), COOH, CON(R₁₀)(R₁₁) et halo ;
R₁₀ et R₁₁, représentant indépendamment H ou un radical alkyle inférieur ;
ou seront des sels de ces mêmes composés.

De préférence, lorsqu'ils seront employés pour l'invention, les composés de formule générale **(II)** seront ceux dans lesquels se retrouvent, indépendamment, les radicaux présentant les caractéristiques suivantes :
- R¹ représentant le radical 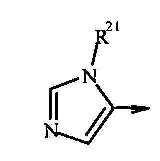 R²¹ représentant un radical aralkyle dont le groupe aryle peut optionnellement être substitué par un ou des radicaux choisis parmi un atome halogène et les radicaux cyano, hydroxy, alkoxy, amino, alkylamino et dialkylamino ;
- R⁴ représentant un radical aryle optionnellement substitué par un ou des radicaux choisis parmi un atome halogène et les radicaux hydroxy, alkoxy, amino, alkylamino et dialkylamino ;
- X représentant un radical alkylène comptant de 1 à 6 atomes de carbone ;
- Y représentant CO ;
- n1 = 1, R¹⁰ étant C, R⁶ représentant H et R¹⁰ et R⁷ formant, pris ensemble, le radical 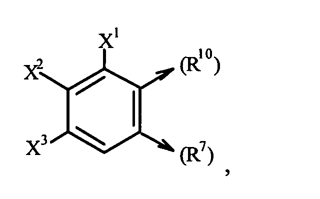
chacun de X¹, X², et X³ représentant, indépendamment, H ou un atome halogène.

De préférence, lorsqu'ils seront employés pour l'invention, les composés de formule générale **(III)** seront ceux dans lesquels se retrouvent, indépendamment, les radicaux présentant les caractéristiques suivantes :
- R¹ représentant OH ou NH₂ ;
- R² représentant alkyle et de préférence méthyle ;
- l'un de R³, R⁴ et R⁵ représentant un atome halogène ;
- R⁶ représentant alkyle et de préférence méthyle ;
- l'un de R⁸ et R⁹ représentant un atome halogène ;
- Y représentant O.

Selon une variante particulièrement préférée de l'invention, les inhibiteurs de la transduction des signaux des protéines G hétérotrimériques seront choisis parmi le groupe composé :
- de la 7-(2-amino-1-oxo-3-thiopropyl)-8-(cyclohexylméthyl)-2-(2-méthylphényl)-5,6,7,8-tétrahydroimidazo[1,2a]pyrazine ; et
- de la 7-(2-amino-1-oxo-3-thiopropyl)-8-(cyclohexylméthyl)-2-phényl-5,6,7,8-tétrahydroimidazo[1,2a]pyrazine ;
et des sels pharmaceutiquement acceptables de ces derniers.

Toujours selon une variante particulièrement préférée de l'invention, les agents anti-cancéreux associés auxdits inhibiteurs de la transduction des signaux des protéines G hétérotrimériques seront choisis parmi le groupe composé :
- de la 1-(2-(1-((4-cyano)phénylméthyl)imidazol-4-yl)-1-oxoéthyl-2,5-dihydro-4-(2-méthoxyphényl)imidazo[1,2c][1,4]benzodiazépine ;
- de la 4-(2-bromophényl)-1-(2-(1-((4-cyano-3-méthoxy)phénylméthyl)-imidazo-5-yl)-1-oxoéthyl)-1,2-dihydro-8-fluoro-imidazo[1,2a][1,4]-benzodiazépine ;
- de la (±)-4-(3-chlorophényl)-6-[(4-chlorophényl)-amino-(1-méthyl-1H-imidazol-5-yl)méthyl]-1-méthyl-2(1H)quinolinone;
- du taxol ;
- de la gemcitabine ;
et des sels pharmaceutiquement acceptables de ces derniers.

Optionnellement, on pourra également faire entrer un composé anti-cancéreux supplémentaire, distinct de l'agent anti-cancéreux associé à l'inhibiteur de la transduction des signaux des protéines G hétérotrimériques, dans la composition du produit de l'invention. De préférence, ledit composé supplémentaire sera choisi parmi le groupe composé :
- de la 1-(2-(1-((4-cyano)phénylméthyl)imidazol-4-yl)-1-oxoéthyl-2,5-dihydro-4-(2-méthoxyphényl)imidazo[1,2c][1,4]benzodiazépine ;
- de la 4-(2-bromophényl)-1-(2-(1-((4-cyano-3-méthoxy)phénylméthyl)-imidazo-5-yl)-1-oxoéthyl)-1,2-dihydro-8-fluoro-imidazo[1,2a][1,4]-benzodiazépine
- de la (±)-4-(3-chlorophényl)-6-[(4-chlorophényl)-amino-(1-méthyl-1H-imidazol-5-yl)méthyl]-1-méthyl-2(1H)quinolinone ;
- du taxol ;
- de la gemcitabine ;
et des sels pharmaceutiquement acceptables de ces derniers.

L'invention a également pour objet une composition pharmaceutique comprenant au moins un des produits selon l'invention.

Les compositions pharmaceutiques comprenant un produit selon l'invention peuvent être sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un produit selon l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection (intramusculaire, sous-cutanée, intraveineuse, etc.), etc. La voie d'administration dépendra bien entendu du type de maladie à traiter.

Les doses d'administration suivantes (journalières, sauf indication contraire) pourront être envisagées pour les différents composés entrant dans la composition d'un produit selon l'invention :
- composé de formule générale **(I)** : de 50 à 200 mg/m² par voie intrapéritonéale ;
- composé de formule générale **(II)** : de 50 à 500 mg/m² per os ;
- taxol : de 1 à 10 mg/kg (voie intrapéritonéale) ou 1 à 3 mg/kg (voie intraveineuse) ;
- cisplatine : de 50 à 80 mg/m² ;
- 5-fluorouracile : de 400 à 800 mg/m² par voie intraveineuse, les administrations étant répétées de 1 à 4 fois par mois ;
- gemcitabine : de 100 à 500 mg/m² par voie intraveineuse (perfusions de 6 h environ).

### Préparation de certains composés entrant dans la composition des produits de l'invention :

### I) Les composés de formule générale (I) sont préparés selon des méthodes analogues à celles décrites dans la demande de brevet PCT WO 97/30053.

Toutefois, les composés suivants ont été décrits ultérieurement dans la demande de brevet WO 00/02881 :
- la 7-(2-amino-1-oxo-3-thiopropyl)-8-(cyclohexylméthyl)-2-(2-méthylphényl)-5,6,7,8-tétrahydroimidazo[1,2a]pyrazine ; et
- la 7-(2-amino-1-oxo-3-thiopropyl)-8-(cyclohexylméthyl)-2-phényl-5,6,7,8-tétrahydroimidazo[1,2a]pyrazine.

### II) Les composés de formule générale (II), dont la 1-(2-(1-((4-cyano)phénylméthyl)imidazol-4-yl)-1-oxoéthyl-2,5-dihydro-4-(2-méthoxyphényl)imidazo[1,2c][1,4]benzodiazépine et la 4-(2-bromophényl)-1-(2-(1-((4-cyano-3-méthoxy)phénylméthyl)imidazo-5-yl)-1-oxoéthyl)-1,2-dihydro-8-fluoroimidazol[1,2a][1,4]-benzodiazépine, sont préparés selon les procédures décrites dans la demande de brevet PCT WO 00/39130.

### C) Les composés de formule générale (III) sont préparés selon les méthodes décrites dans la demande de brevet PCT WO 97/21701.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

Afin d'illustrer l'utilité de l'invention, on étudiera l'effet d'un traitement sur une lignée tumorale de cellules humaines pancréatiques Mia-Paca2 par la 7-(2-amino-1-oxo-3-thiopropyl)-8-(cyclohexylméthyl)-2-(2-méthylphényl)-5,6,7,8-tétrahydroimidazo[1,2a]pyrazine ou la 7-(2-amino-1-oxo-3-thiopropyl)-8-(cyclohexylméthyl)-2-phényl-5,6,7,8-tétrahydroimidazo[1,2a]pyrazine (décrites dans la demande PCT WO 00/02881) en association avec différents agents anti-cancéreux.

Par convention, les produits inhibant la transduction des signaux des protéines G hétérotrimériques utilisés dans les tests seront identifiés par la lettre A, et les autres agents anti-cancéreux utilisés dans les tests par la lettre B.

### 1) Procédures

### Matériel

Les composés suivants (préparés selon les méthodes décrites précédemment) entrent dans la composition des produits testés :
- la 7-(2-amino-1-oxo-3-thiopropyl)-8-(cyclohexylméthyl)-2-(2-méthylphényl)-5,6,7,8-tétrahydroimidazo[1,2a]pyrazine (composé **A**_{**1**}) ;
- la 7-(2-amino-1-oxo-3-thiopropyl)-8-(cyclohexylméthyl)-2-phényl-5,6,7,8-tétrahydroimidazo[1,2a]pyrazine (composé **A**_{**2**}) ;
- 1-(2-(1-((4-cyano)phénylméthyl)imidazol-4-yl)-1-oxoéthyl-2,5-dihydro-4-(2-méthoxyphényl)imidazo[1,2c][1,4]benzodiazépine (composé **B**_{**1**}) ;
- le taxol (composé **B**_{**2**}) ;
- la gemcitabine (composé **B**_{**3**}) ;
- la (±)-4-(3-chlorophényl)-6-[(4-chlorophényl)-amino-(1-méthyl-1H-imidazol-5-yl)méthyl]-1-méthyl-2(1H)quinolinone (composé **B**_{**4**}) ;
- la 4-(2-bromophényl)-1-(2-(1-((4-cyano-3-méthoxy)phénylméthyl)-imidazo-5-yl)-1-oxoéthyl)-1,2-dihydro-8-fluoro-imidazo [1,2a][1,4]-benzodiazépine (composé **B**_{**5**}).

### Lignée cellulaire

La lignée cellulaire Mia-Paca2 (cellules humaines de cancer du pancréas) a été acquise auprès de American Tissue Culture Collection (Rockville, Maryland, USA).

### Mesure de la prolifération cellulaire in vitro

Les cellules Mia-Paca2 (1500 cellules/puits) sont cultivées en plaques 96 puits pré-coatées par le polyhema (Sigma) qui autorise uniquement la croissance des cellules présentant un phénotype tumorigène.

Au jour 0, ces cellules sont ensemencées dans 90 µl de milieu de Eagle modifié par Dulbecco (Gibco-Brl, Cergy-Pontoise, France) complété avec 10% de sérum foetal de veau inactivé par chauffage (Gibco-Brl, Cergy-Pontoise, France ), 50000 unités/l de pénicilline et 50 mg/l streptomycine (Gibco-Brl, Cergy-Pontoise, France), et 2 mM de glutamine (Gibco-Brl, Cergy-Pontoise, France).
Les cellules ont été traitées avec des concentrations croissantes de deux produits seuls ou en association de manière matricielle, soit : au jour 1, le premier produit pendant 96 heures avec au jour 2 le second produit pendant 72 heures. Selon la méthode α, l'inhibiteur de la transduction des signaux des protéines G hétérotrimériques auquel l'agent anti-cancéreux est associé est administré *avant* ce dernier, alors que selon la méthode β, il l'est *après*.
A la fin de cette période, la quantification de la prolifération cellulaire est évaluée par test colorimétrique en se basant sur le clivage du sel de tétrazolium WST1 par les déhydrogénases mitochondriales dans les cellules vivantes conduisant à la formation de formazan (Boehringer Mannheim, Meylan, France). Ces tests sont effectués en double avec 4 déterminations pour chaque produit seul et pour chaque association testée. Ceci permet de déterminer le nombre de cellules vivantes à la fin de chaque traitement, soit la valeur observée. La valeur calculée des cellules vivantes pour chaque traitement correspond à la multiplication des valeurs observées des effets des produits séparés. Ces valeurs observées et calculées sont comparées pour chaque association. Quand la valeur observée de cellules vivantes est inférieure à la valeur calculée des cellules vivantes, une synergie est considérée. Quand la valeur observée est égale à la valeur calculée, une additivité est considérée. Quand la valeur observée est supérieure à la valeur calculée, un antagonisme est considéré.

### 2) Résultats :

Les résultats obtenus sont reportés dans les tableaux figurant ci-après.

Les résultats reportés dans les tableaux I, II, III, IV et IV montrent que les produits comprenant le composé **A**_{**1**} en association avec le composé **B**_{**1**}**,** le composé **B**_{**2**} ou le composé **B**_{**3**} ou ceux comprenant le composé **A**_{**2**} en association avec le composé **B**_{**4**} ou le composé **B**_{**5**} sont capables d'inhiber la prolifération *in vitro* des cellules tumorales humaines Mia-Paca2. L'effet combiné de l'association, évalué par la méthode décrite dans Cote, S. et Momparler, R.L., *Anticancer Drugs* (1993), **4**, 327-333, permet de constater une synergie pour les associations **A**_{**1**} + **B**_{**1**}**, A**_{**1**} + **B**_{**2**}**, A**_{**1**} + **B**_{**3**}**, A**_{**2**} + **B**_{**4**} et **A**_{**2**} + **B**_{**5**}**.**

**Tableau I**

| Doses du composé B₁ | Composé B₁ seul | *Valeurs observées (méthode α - n=4)* Composé A₁ (20 µM) + composé B₁ | *Valeurs calculées* Composé A₁ (20 µM) + composé B₁ |
|---|---|---|---|
| 0 µM | 100 | 51 ± 8,9 | |
| 0,04 µM | 94 ± 2,3 | 39 ± 8,4 | 47 ± 7,1 |
| 0,2 µM | 80 ± 4,2 | 23 ± 5,9 | 38 ± 4,9 |
| 1 µM | 68 ± 2,3 | 23 ± 6,5 | 34 ± 5,0 |

**Tableau II**

| Doses du composé B₂ | Composé B₂ seul | *Valeurs observées (méthode α - n=3)* Composé A₁ (20 µM) + composé B₂ | *Valeurs calculées* Composé A₁ (20 µM) + composé B₂ |
|---|---|---|---|
| 0 nM | 100 | 49 ± 7,7 | |
| 0,8 nM | 100 ± 5,8 | 34 ± 2,4 | 48 ± 6,7 |
| 4 nM | 100 ± 0,3 | 32 ± 2,2 | 49 ± 7,5 |
| 20 nM | 60 ± 10,6 | 17 ± 4,5 | 30 ± 7,2 |

**Tableau III**

| Doses du composé B₃ | Composé B₃ seul | *Valeurs observées (méthode α - n=3)* Composé A₁ (20 µM) + composé B₃ | *Valeurs calculées* Composé A₁ (20 µM) + composé B₃ |
|---|---|---|---|
| 0 nM | 100 | 65 ± 7,3 | |
| 4 nM | 95 ± 9,8 | 48 ± 2,1 | 59 ± 1,3 |
| 20 nM | 72 ± 11,5 | 27 ± 6,8 | 45 ± 4,0 |
| 100 nM | 62 ± 1,9 | 30 ± 4,0 | 40 ± 3,1 |

**Tableau IV**

| Doses du composé B₄ | Composé B₄ seul | *Valeurs observées (méthode β- n=2)* Composé A₂ (20 µM) + composé B₄ | *Valeurs calculées* Composé A₂ (20 µM) + composé B₄ |
|---|---|---|---|
| 0 nM | 100 | 42 ± 11,0 | |
| 40 nM | 104 ±2,0 | 28 ± 3,0 | 44 ± 12,0 |
| 0,2 µM | 90 ±9,0 | 13 ± 2,0 | 37 ± 6,0 |
| 1 µM | 72 ± 3 | 14 ± 1,0 | 30 ± 7,0 |

**Tableau V**

| Doses du composé B₅ | Composé B₅ seul | *Valeurs observées (méthode β - n=3)* Composé A₂ (20 µM) + composé B₅ | *Valeurs calculées* Composé A₂ (20 µM) + composé B₅ |
|---|---|---|---|
| 0 µM | 100 | 44 ± 6,4 | |
| 0,2 µM | 86 ± 0,9 | 17 ± 2,7 | 38 ± 5,2 |
| 1 µM | 63 ± 2,6 | 11 ± 1,4 | 27 ± 3,0 |

## Revendications

1. Produit comprenant au moins un inhibiteur de la transduction des signaux des protéines G hétérotrimériques choisi parmi le groupe constitué par les composés de formule générale **(I**_{**A**}**)** 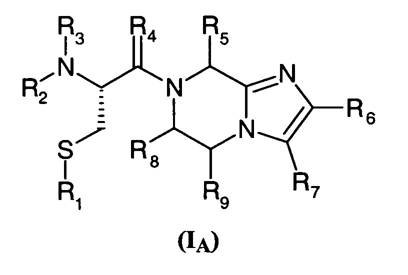 dans laquelle :
R₁ représente un radical alkyle de 1 à 6 atomes de carbone ;
R₂ et R₃ représentent H ;
R₄ représente O ;
R₅ représente H, ou l'un des radicaux alkyle de 1 à 6 atomes de carbone, cycloalkyle, cycloalkylalkyle dont le radical alkyle compte de 1 à 6 atomes de carbone, arylsulfonylalkyle dont le radical alkyle compte de 1 à 6 atomes de carbone et aralkoxyalkyle dont les radicaux alkoxy et alkyle comptent chacun de 1 à 6 atomes de carbone, ces radicaux pouvant éventuellement être substitués par des radicaux choisis parmi le groupe composé d'un radical alkyle de 1 à 6 atomes de carbone et -O-R₁₀ ;
R₆ et R₇ représentent indépendamment H ou un radical aryle éventuellement substitué par des radicaux choisis parmi le groupe composé des radicaux OH et alkyle ou alkoxy de 1 à 6 atomes de carbone ;
R₈ et R₉ représentent H ;
et R₁₀ et R₁₁, représentent indépendamment H ou un radical alkyle de 1 à 6 atomes de carbone ;
et les sels pharmaceutiquement acceptables des composés de formule générale **(I)**
en association avec au moins un autre agent anti-cancéreux choisi parmi les agents alkylants, pour une utilisation thérapeutique simultanée, séparée ou étalée dans le temps, dans le traitement du cancer.

2. Produit selon la revendication 1, **caractérisé en ce que** l'agent alkylant est le cis-platine.

3. Produit selon la revendication 1 ou 2, **caractérisé en ce que** les composés de formule générale **(I**_{**A**}**)** et leurs sels pharmaceutiquement acceptables sont choisis parmi le groupe constitué par :
• la 7-(2-amino-1-oxo-3-thiopropyl)-8-(cyclohexylméthyl)-2-(2-méthylphényl)-5,6,7,8-tétrahydroimidazo[1,2a]pyrazine ;
• la 7-(2-amino-1-oxo-3-thiopropyl)-8-butyl-2-(2-méthoxyphényl)-5,6,7,8-tétrahydroimidazo[1,2a]pyrazine ;
• la 7-(2-amino-1-oxo-3-thiopropyl)-8-(cyclohexylméthyl)-2-phényl-5,6,7,8-tétrahydroimidazo[1,2a]pyrazine ;
• la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
• la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
• la 7-(2-amino-1-oxo-3-thiopropyl)-2-(2-méthoxyphényl)-8-(phénoxyéthyl)-5,6,7,8-tétrahydroimidazo[1,2a]pyrazine ;
• la 7-(2-amino-1-oxo-3-thiopropyl)-2-(2-méthoxyphényl)-8-(phénoxyéthyl)-5,6,7,8-tétrahydro-imidazo[1,2a]pyrazine, ou sa forme dimérique ;
• et la 7-(2-amino-1-oxo-3-thiopropyl)-2-(2-méthoxyphényl)-8-(phénylsulfonyléthyl)-5,6,7,8-tétrahydro-imidazo[1,2a]pyrazine ;
et des sels pharmaceutiquement acceptables de ces derniers.

4. Produit selon la revendication 3, **caractérisé en ce que** les composés de formule générale **(I**_{**A**}**)** et leurs sels pharmaceutiquement acceptables sont choisis parmi le groupe constitué par :
- de la 7-(2-amino-1-oxo-3-thiopropyl)-8-(cyclohexylméthyl)-2-(2-méthylphényl)-5,6,7,8-tétrahydroimidazo[1,2a]pyrazine ; et
- de la 7-(2-amino-1-oxo-3-thiopropyl)-8-(cyclohexylméthyl)-2-phényl-5,6,7,8-tétrahydroimidazo[1,2a]pyrazine ;
et des sels pharmaceutiquement acceptables de ces derniers.

5. Produit selon l'une des revendications 1 à 4, **caractérisé en ce qu'**il comprend en outre un composé anti-cancéreux supplémentaire, ledit composé étant choisi parmi le groupe composé :
- de la 1-(2-(1-((4-cyano)phénylméthyl)imidazol-4-yl)-1-oxoéthyl-2,5-dihydro-4-(2-méthoxyphényl)imidazo[1,2c][1,4]benzodiazépine ;
- de la 4-(2-bromophényl)-1-(2-(1-((4-cyano-3-méthoxy)phénylméthyl)-imidazo-5-yl)-1-oxoéthyl)-1,2-dihydro-8-fluoro-imidazo[1,2a][1,4]-benzodiazépine ;
- de la (±)-4-(3-chlorophényl)-6-[(4-chlorophényl)-amino-(1-méthyl-1H-imidazol-5-yl)méthyl]-1-méthyl-2(1H)quinolinone ;
- du taxol ;
- de la gemcitabine ;
et des sels pharmaceutiquement acceptables de ces derniers.

6. Composition pharmaceutique comprenant au moins un produit selon l'une des revendications 1 à 5.
